# EUROPEAN PATENT SPECIFICATION

(11) **EP 2 578 234 B1**
(45) Date of publication and mention of the grant of the patent: **12.07.2017**
(21) Application number: 11786640.0
(22) Date of filing: 24.05.2011
(51) Int. Cl.: A61K 47/32, A61K 9/00, A61K 38/28, A61M 1/36, A61M 5/172

(54) **MEDICINE ADMINISTERING DEVICE COMPRISING A SUGAR REPSONSIVE GEL**
MEDIKAMENTENVERABREICHUNGSVORRICHTUNG ENTHALTEND EIN AUF ZUCKER REAGIERENDES GEL
DISPOSITIF D'ADMINISTRATION DE MÉDICAMENTS CONTENANT UN GEL SENSIBLE AU SUCRE

(30) Priority: 17.09.2010 JP 2010208796; 26.05.2010 US 348334 P
(43) Date of publication of application: 10.04.2013
(73) Proprietor: National Institute for Materials Science, Tsukuba-shi, Ibaraki 305-0047 (JP)
(72) Inventor: MATSUMOTO, Akira, Tokyo 113-8654 (JP); ISHII, Takehiko, Tokyo 113-8654 (JP); KATAOKA, Kazunori, Tokyo 113-8654 (JP); MIYAHARA, Yuji, Tsukuba-shi Ibaraki 305-0047 (JP)
(74) Representative: Le Guen-Maillet
(86) International application number: PCT/JP2011/061869
(87) International publication number: WO 2011/148941

(56) References cited:
- WO-A1-2004/081624
- WO-A1-2011/083711
- JP-A- 11 322 761
- JP-A- 61 087 565
- JP-A- 2006 505 305
- US-A1- 2009 021 697
- AKIRA MATSUMOTO ET AL: "A totally synthetic glucose responsive gel operating in physiological aqueous conditions", CHEMICAL COMMUNICATIONS, vol. 46, no. 13, 1 January 2010 (2010-01-01), page 2203, XP055105757, ISSN: 1359-7345, DOI: 10.1039/b920319b
- AKIRA MATSUMOTO ET AL: "Glucose-sensitive field effect transistor using totally synthetic compounds", JOURNAL OF SOLID STATE ELECTROCHEMISTRY ; CURRENT RESEARCH AND DEVELOPMENT IN SCIENCE AND TECHNOLOGY, SPRINGER, BERLIN, DE, vol. 13, no. 1, 6 July 2008 (2008-07-06), pages 165-170, XP019635334, ISSN: 1433-0768
- AKIRA MATSUMOTO ET AL: "Swelling and Shrinking Kinetics of Totally Synthetic, Glucose-Responsive Polymer Gel Bearing Phenylborate Derivative as a Glucose-Sensing Moiety", MACROMOLECULES, vol. 37, no. 4, 1 February 2004 (2004-02-01), pages 1502-1510, XP055105754, ISSN: 0024-9297, DOI: 10.1021/ma035382i
- D EDDINGTON: "Flow control with hydrogels", ADVANCED DRUG DELIVERY REVIEWS, vol. 56, no. 2, 10 February 2004 (2004-02-10), pages 199-210, XP055105833, ISSN: 0169-409X, DOI: 10.1016/j.addr.2003.08.013
- KABILANA, K. ET AL.: 'Holographic glucose sensors' BIOSENSORS AND BIOELECTRONICS vol. 20, no. 8, 2005, pages 1602 - 1610, XP027619502
- MATSUMOTO, A. ET AL.: 'Glucose-Responsive Polymer Gel Bearing Phenylborate Derivative as a Glucose-Sensing Moiety Operating at the Physiological pH' BIOMACROMOLECULES vol. 5, no. 3, 2004, pages 1038 - 1045, XP008159372
- YAN, J. ET AL.: 'The relationship among pKa, pH, and binding constants in the interactions between boronic acids and diols-it is not as simple as it appears' TETRAHEDRON vol. 60, no. 49, 2004, pages 11205 - 11209, XP004617676

## Description

### TECHNICAL FIELD

This invention relates to a sugar-responsive gel and a medicine administering device.

### BACKGROUND OF THE INVENTION

Methacrylamide phenylboronic acid (e.g., refer to patent document 1), 3-acrylamide-6-hexafluoropropyl phenylboronic acid (e.g., refer to patent document 2), N-(4'-vinylbenzyl)-4-phenylboronic acid carboxyamide (e.g., refer to patent document 3), 4-(1',6'-dioxo-2',5'-diaza-7'-octenyl) phenylboronic acid and the like are known as conventional phenylboronic acid derivatives (hereinafter referred to as PBAs) having a polymerizable unsaturated bond.

Patent Application JPS 61 087565 discloses an implantable insulin administration device. However, the device is not an autonomous administration device. The pump and auxiliary pump must be compressed by the patent's finger to cause the fluid within the pump to be expelled through the tubing sectionand the check valve.

Patent Application JP 2006 505305 discloses an implantable artificial pancreas equipping a pump which works by electronic power and a glucose monitoring device utilizing glucose oxidase. It dos not discloses a device having a glucose responsive gel including phenylboronic acid derivatives of the present invention. It is noted that glucose oxidase is not stable for use to a biological sample for a long time.

The scientific publication « Flow control with hydropels », D.T. Eddington, D.J. Beebe, 2004, is a review article of responsive hydrogel actuators for flow control in drug delivery devices. It describes a system containing glucose oxidase and catalase for a glucose sensor similar to JP 2006 505305 i.e., the volume of hydrogel is responsive to a pH change. However, it neither discloses nor suggests a glucose responsive gel containing phenylboronic acid derivatives and an autonomous device including such a gel for insulin injection

### PRIOR ART DOCUMENTS

### PATENT DOCUMENTS

Patent document 1: Japanese unexamined patent application publication No. H3-204823.
Patent document 2: Japanese unexamined patent application publication No. H5-301880.
Patent document 3: Japanese unexamined patent application publication No. H5-262779.
Patent document 4: Japanese unexamined patent application publication No. H11-322761.

### SUMMARY OF THE INVENTION

### PROBLEMS TO BE SOLVED BY THE INVENTION

Any of these conventional phenylboronic acid derivatives, however, has not ever simultaneously satisfied all of the following three properties: (1) having high hydrophilicity; (2) having low pKa (a value represented by -log₁₀Ka, herein Ka denotes an acid dissociation constant); and (3) having a polimerizable unsaturated bond. Accordingly, when they are polymerized in water as a solvent, these conventional phenylboronic acid derivatives cannot be borne in a polymer in a sufficient amount, or cannot work fully as a sugar-responsive gel capitalizing on the Lower Critical Solution Temperature (LCST), because the polymerized polymer and the aqueous gel have a high hydrophobicity.

While, since a phenylboronic acid compound has a capability of reversible covalent binding to sugar molecules such as glucose, there has been developed a sugar-responsive gel that releases insulin autonomously in response to a glucose concentration, utilizing such properties of the phenylboronic acid compound.

Accordingly, the present invention has been accomplished, considering the properties described above, and an object of the present invention is to propose a sugar-responsive gel and a medicine administering device having pKa more suitable than conventional ones for the use in a body environment and capable of releasing a medicine autonomously.

### MEANS TO SOLVE THE PROBLEMS

A first aspect of the present invention for solving the problems is characterized by a device for administering a medicine in response to a blood-sugar level of a subject to be administered with a medicine, comprising: a sugar-responsive gel, comprising a gel body and a dehydrated shrunken layer enclosing the gel body, wherein a medicine is contained in the gel body, wherein the gel body and the dehydrated shrunken layer are formed by a gel composition containing a gelling agent, a cross-linking agent and a phenylboronic acids monomer represented by the following formula (1): 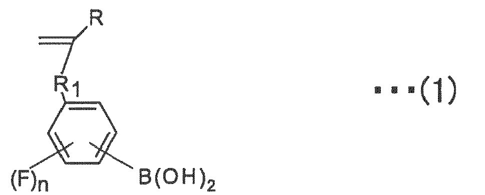 wherein:
R is H or CH₃;
F is independently present;
n is 1, 2, 3 or 4; and
R₁ is a divalent linking group.
an introducing unit for introducing the blood of the subject to be administered with the medicine into the sugar-responsive gel; and
a releasing unit for releasing the medicine into the subject to be administered with the medicine based on a change in the sugar-responsive gel in response to a blood-sugar level of the blood,
wherein the introducing unit and the releasing unit are an indwelling needle whose tip is inserted into the administration site to be held therein, and the sugar-responsive gel is filled into the indwelling needle.

The invention also relates to a device for administering a medicine in response to a blood-sugar level of a subject to be administered with a medicine, comprising:
a sugar-responsive gel, comprising a gel body, wherein a medicine is contained in the gel body, wherein the gel body is formed by a gel composition containing a gelling agent, a cross-linking agent and a phenylboronic acids monomer represented by the following formula (1): 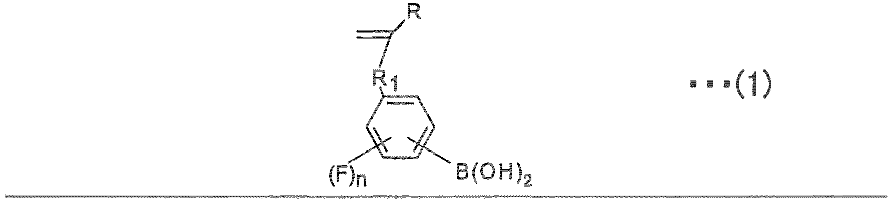 wherein:
   R is H or CH₃;
   F is independently present;
   n is 1, 2, 3 or 4; and
   R₁ is a divalent linking group;
an introducing unit for introducing the blood of the subject to be administered with the medicine into the sugar-responsive gel;
a releasing unit for releasing the medicine into the subject to be administered with the medicine based on a change in the sugar-responsive gel in response to a blood-sugar level of the blood;
an indwelling needle with a backflow prevention valve whose tip is inserted into the administration site to be held therein; and
a filling unit filled with the medicine and communicated with the indwelling needle with the backflow prevention valve,
wherein the releasing unit releases the medicine into the administration site from the indwelling needle with the backflow prevention valve in such a manner that the medicine in the filling unit is pushed out to the indwelling needle with the backflow prevention valve by an expansion pressure generated in response to the blood-sugar level of the blood introduced by the introducing unit.

Advantageously, the medicine administering device comprises a supplying unit for supplying the medicine to the sugar-responsive gel.

### EFFECT OF THE INVENTION

According to the present invention, the present invention can work under the condition of pKa 7.4 or less, comprising a component that can release a medicine depending on an elevation of the sugar concentration, and, having pKa more suitable than conventional ones for the use in a body environment and capable of administering a medicine autonomously.

### BRIEF DESCRIPTION OF THE DRAWINGS

Fig. 1 is a synthetic scheme showing the synthesis of a phenylboronic acids monomer contained in a sugar-responsive gel of the present invention.
Fig. 2 is a graph showing a relationship between apparent pKas and glucose concentrations.
Fig. 3 is a graph showing a relationship between the swelling degree and temperature of the sugar-responsive gel, along with photographs of gels in certain glucose concentrations at the temperature of 37°C (degrees Celsius).
Fig. 4 is a graph showing a relationship (1) between the glucose concentration and the fluorescence intensity.
Fig. 5 is a graph showing a relationship (2) between the glucose concentration and the fluorescence intensity.
Fig. 6 shows transmission light images of the sugar-responsive gel in a certain glucose concentration.
Fig. 7 is a schematic overall configuration showing an insulin administering device of a first embodiment.
Fig. 8 is a schematic overall configuration showing the insulin administering device of a second embodiment.
Fig. 9 is a schematic overall configuration showing the insulin administering device of a third embodiment.

### Explanation of Numerals

- 10, 20, 30: Insulin administering device (medicine administering device)
- 13, 23, 41: Sugar-responsive gel
- 12: Supplying unit
- 14: Enclosing unit (releasing unit)
- 22: Indwelling needle (releasing unit, introducing unit)
- 31: Insulin filling unit having a pumping function (filling unit)
- 32: Indwelling needle with backflow prevention valve (releasing unit)
- 36: Introducing unit

### MODE FOR CARRYING OUT THE INVENTION

Embodiments of the invention are described in details hereinbelow with reference to the drawings.

(1) A phenylboronic acids monomer to be contained in a sugar-responsive gel of the present invention:
   A phenylboronic acids monomer to be contained in a sugar-responsive gel of the present invention is represented by the following formula (5): 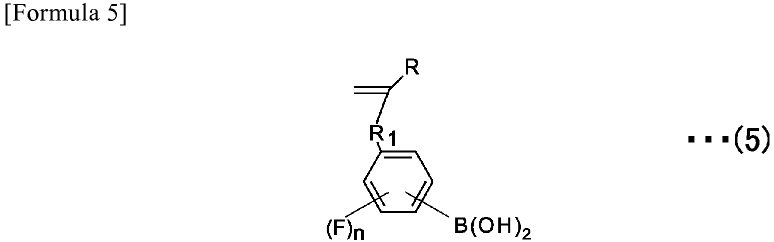 (wherein R is H or CH₃, F is independently present, n is 1, 2, 3 or 4, and R₁ is a divalent linking group).

The divalent linking group represented by R₁ comprises one or more bonds selected from the group consisting of carbamoyl bond, amide bond, alkyl bond, ether bond, ester bond, thioester bond, thioether bond, sulfonamide bond, urethane bond, sulfonyl bond, imine bond, urea bond, thiourea bond and the like.

As described above, the phenylboronic acids monomer has a structure wherein one or more of fluorines are substituted for hydrogens on the phenyl ring of phenylboric acid group. Also, the phenylboronic acids monomer has a structure wherein the linking group R₁ is bound to the phenyl ring and a carbon in unsaturated bond is bound to the linking group R₁.

The phenylboronic acids monomer has a high hydrophilicity, and its pKa can be controlled to the values of 7.4 or less in the physiological level by fluorination of the phenyl ring. Further, this phenylboronic acids monomer can acquire the abilities not only to recognize a sugar in the physiological environments, but also to copolymerize with a gelling agent and a linking agent described below by an unsaturated bond, and thus can form a sugar-responsive gel.

As an embodiment, in phenylboronic acid monomer represented by the formula (5), if n is 1 and one fluorine is substituted for a hydrogen on the phenyl ring, F and B(OH)₂ to be substituted may be in any of ortho, metha and para positions.

Under this condition, if carbamoyl group is applied to the linking group R₁, the above formula (5) is represented by the following formula (6): 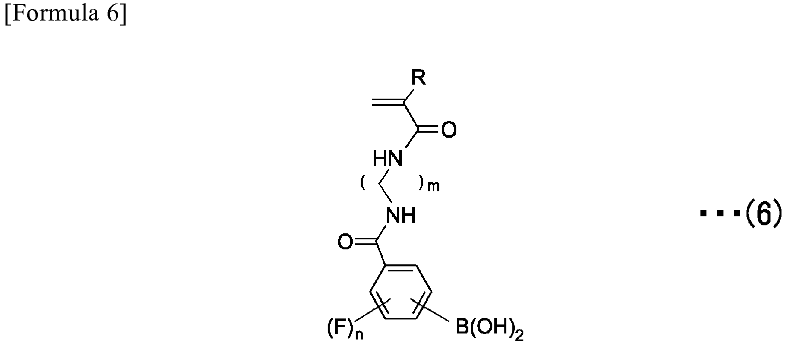 (wherein m is an integer of 0, or 1 or more).

As described above, a phenylboronic acids monomer has the structure comprising a fluorophenyl boronic acid group wherein one or a plurality of fluorines are substituted for hydrogens on the phenyl ring, and the carbon of amide group is bound to the phenyl ring.

In addition, if m is 0, the phenylboronic acids monomer has the structure wherein nitrogen of the amide group directly binds to nitrogen of other amide group to contain therein an acrylamide or methacrylamide structure comprising the part of this other amide group. While, if m is one or more, the phenylboronic acids monomer has the structure wherein nitrogen of the amide group binds to nitrogen of other amide group via one or a plurality of carbons to contain therein the acrylamide or methacrylamide structure comprising the part of this other amide group. Additionally, if m is one or more, the pKa of the phenylboronic acids monomer can be controlled to a lower value compared with the value obtained if m is 0. Further, even such phenylboronic acids monomer can achieve similar effect to that of the phenylboronic acids monomer represented by the above formula (5).

One embodiment of the formula (6) described above is a phenylboronic acids monomer, wherein n is 1, a fluorine is substituted for one hydrogen on the ring of phenylboronic acid group, m is 2 (spacer carbons are two), and its formula is represented by the following formula (7) (wherein R is H or CH₃):

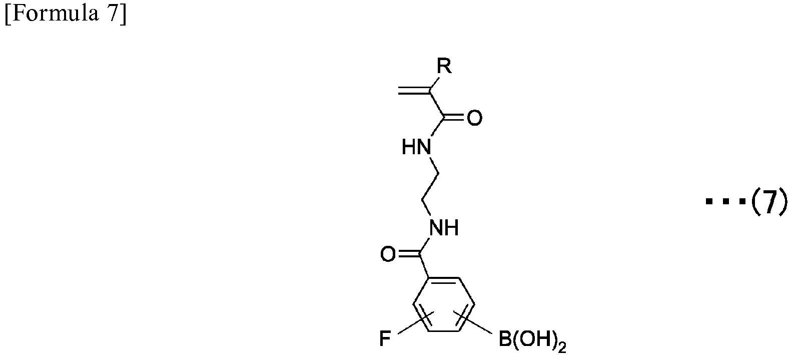

### (2) Phenylboronic acids polymer

Here, a phenylboronic acids polymer polymerized among the phenylboronic acids monomer, represented by the formula (5) mentioned above, is represented by the following formula (8):

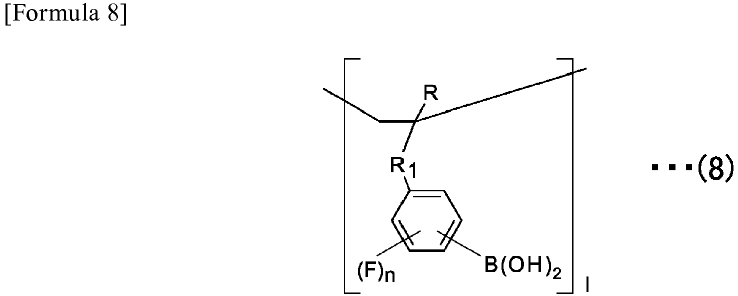

(R is H or CH₃, F is independently present, n is 1, 2, 3 or 4, 1 is an integer of two or more, and R₁ is a divalent linking group).

In addition, as one embodiment of the divalent linking group, if a linking group comprising carbamoyl bond is applied, the above formula (8) is represented by the following formula (9) (wherein m is an integer of 0, or 1 or more):

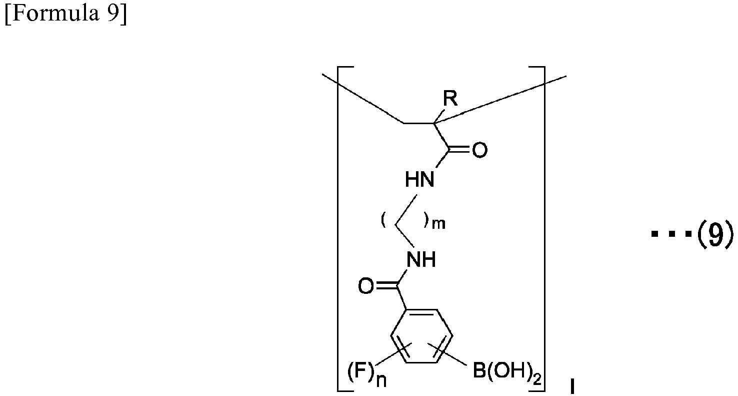

In addition, by polymerization among the phenylboronic acids monomers represented by the above-described formula (7), a phenylboronic acids polymer represented by the following formula (10) can be obtained (wherein R is H or CH₃, 1 is an integer of two or more):

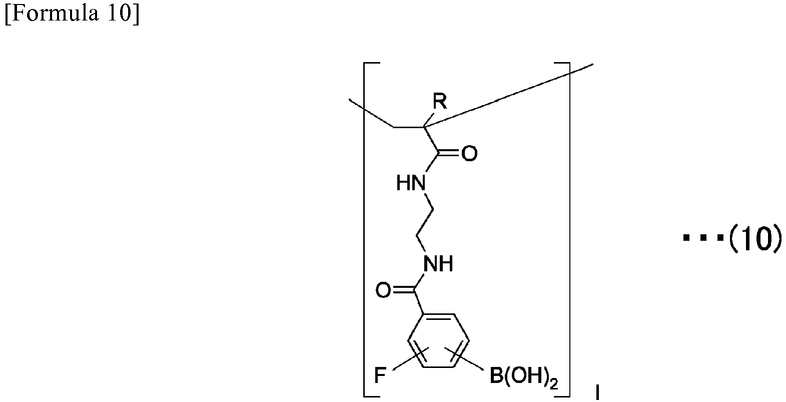

### (3) Method for producing the phenylboronic acids monomer

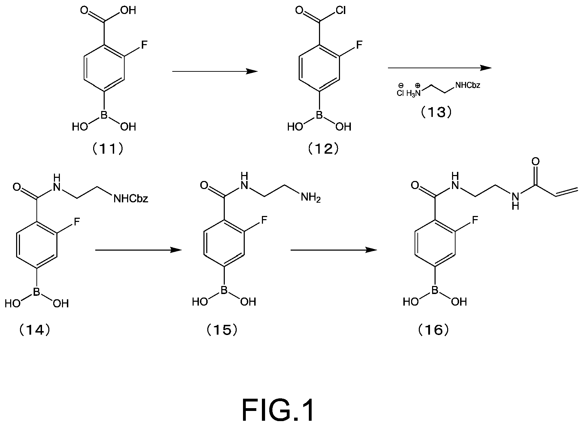

Here, the phenylboronic acids monomer represented by the above formula (7) is produced according to the synthetic scheme as shown in Fig. 1. At first, carboxy-fluorophenylboronic acid represented by the formula (11) in Fig. 1 is refluxed after addition of thionyl chloride, and then acid chloride compound represented by the formula (12) is synthesized.

Next, the acid chloride compound represented by the above formula (12) is dissolved in tetrahydrofuran (THF), triethylamine (TEA) is added thereto as a base catalyst, and reacted by addition of the compound represented by the following formula (13), then an intermediate represented by the formula (14) (Fig.1) is synthesized.

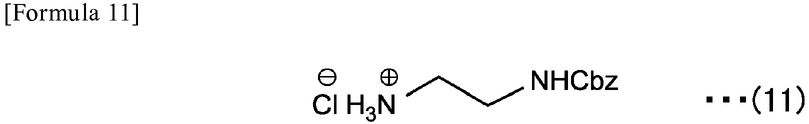

Subsequently, in the presence of hydrogen gas, an intermediate represented by the formula (15) (Fig.1) is synthesized by reduction reaction of the intermediate represented by the formula (14) with palladium/carbon (Pd/C) catalyst. Then, by Schotten-Baumann method, the phenylboronic acids monomer of the present invention represented by the formula (16) (Fig.1) can be synthesized by mixing the intermediate represented by the formula (15) with acryloyl chloride.

### (4) Verification of the phenylboronic acids monomer

### (4-1) The phenylboronic acids monomer to be contained in the sugar-responsive gel of the present invention

Subsequently, according to the synthetic scheme shown in Fig.1, the phenylboronic acids monomer to be contained in the sugar-responsive gel of the present invention was synthesized. In particular, 4-(2-acrylamide ethylcarbamoyl)-3-fluorophenylboronic acid (hereinafter referred to as sample 1) was synthesized according to the following procedures as an example of the phenylboronic acids monomer.

At first, 27 mmol of carboxyfluorophenylboronic acid (formula (11)) was added with 50 mL of thionyl chloride, and refluxed at 90°C (degrees Celsius) in an oil bath, and then a solution was produced. Subsequently, the redundant thionyl chloride was removed from the reaction mixture, which was then dissolved in 90 mL of tetrahydrofurane (THF), added with 40 mmol of the compound represented by the above formula (13), further added with triethylamine (TEA) 200 mmol in an ice water bath, and then the mixture was stirred at room temperature for one day.

The product solution obtained in this manner was added with a diluted hydrochloric acid solution saturated with sodium chloride salt, and subjected to the procedures for washing and separating the solution, and then THF was removed. The residue was dissolved in 400 mL of ethanol, and added with 1 g of 10% palladium carbon catalyst, and subjected to hydrogen reduction reaction under the condition of 40°C (degrees Celsius). Then, the palladium carbon catalyst was filtered out therefrom, and the intermediate represented by the formula (15) (Fig.1) was obtained from the filtrated solution. Subsequently, the obtained intermediate was added with 50 mmol of acryloyl chrolide and 150 mL of a buffer solution of a carbonate salt (100 mM, pH 10), and stirred. In this way, sample 1 as a working example was obtained.

### (4-2) A first comparative example and a second comparative example

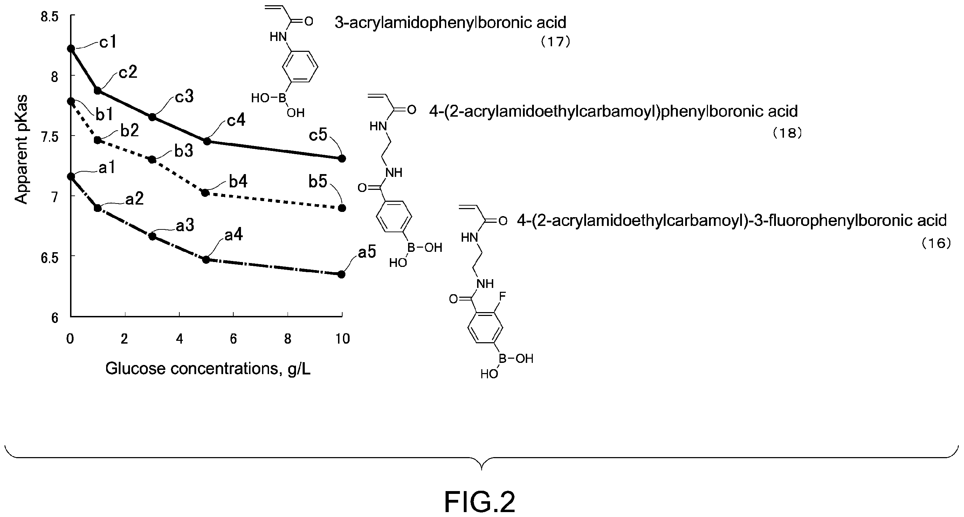

Subsequently, as a first comparative example, 3-acrylamidephenyl boronic acid (Wako Pure Chem. Ltd., hereinafter referred to as comparative sample 1) represented by the formula (17) shown in Fig.2 was prepared.

Subsequently, as a second comparative example, 4-(2-acrylamideethylcarbamoyl) phenylboronic acid (hereinafter referred to as comparative sample 2) represented by the formula (18) shown in Fig.2 was prepared. The comparative sample 2 was prepared, according to the identical procedures to those for the sample 1, from carboxyphenylboronic acid as a starting raw material instead of carboxyfluorophenyl boronic acid used in the sample 1.

### (4-3) Measurement of pKa

Next, for the sample 1, the comparative sample 1 and the comparative sample 2, the relationships between glucose concentrations and apparent changes in pKa were derived by acid-base titration in the conditions of various glucose concentrations (0 g/L, 1 g/L, 3 g/L, 5 g/L and 10 g/L).

The results were shown in Fig. 2. As shown in Fig. 2, samples a1 to a5 using the phenylboronic acids monomer contained in the sugar-responsive gel of the present invention (sample 1) showed lower levels of pKa than 7.4 of the physiological level, and it was observed that the degrees of decrease in pKa according to the increase in concentration of glucose were remarkably superior to samples b1 to b5 and samples c1 to c5 using the conventional comparative samples 1 and 2.

### (5) Sugar-responsive gel of the present invention

Next is a description of the sugar-responsive gel of the present invention containing the phenylboronic acids monomer described in "(1) A phenylboronic acids monomer to be contained in a sugar-responsive gel of the present invention", and the phenylboronic acids polymer described in "(2)Phenylboronic acids polymer". Such sugar-responsive gel can be produced from a gel composition comprising a gelling agent having a property (biocompatibility) that does not cause a toxic effect or an adverse effect to an in vivo biological function, the above-described phenylboronic acids polymer represented by the formula (5), and a cross-linking agent.

Then, the sugar-responsive gel can be produced by the procedures shown as follows: At first, a gel composition is prepared so as to comprise a gelling agent which is to form a main chain of the gel, the phenylboronic acids monomer represented by the above-described formula (5), and a cross-linking agent in a certain molar ratio described later. Subsequently, they are mixed and reacted with one another, thus producing a gel body in a gel state. Then, a medicine is diffused into the gel body by immersion of the gel body in a phosphate buffer containing a certain concentration of insulin, etc. Next, the gel body picked up from the phosphate buffer is immersed in hydrochloric acid for a certain period of time, thus forming a thin dehydrated shrunken layer on the surface of the gel body (hereinafter referred to as a skin layer), and thus the sugar-responsive gel loading the medicine can be produced.

In that case, the gel composition can be prepared so as to comprise the gelling agent, the above-described phenylboronic acids monomer represented by the formula (5), and the cross-linking agent, in a molar mixing ratio of reaction materials (gelling agent / phenylboronic acids monomer / cross-linking agent) being, for example, 92.5 / 7.5 / 1. According to the embodiment of the present invention, the description was given about the preparation in the molar mixing ratio of reaction materials (gelling agent /phenylboronic acids monomer /cross-linking agent) being 92.5 / 7.5 / 1. The present invention, however, is not limited thereto. Other molar ratios may be applicable, provided that a gel body formed by a gel composition comprising the gelling agent, the phenylboronic acids monomer and the cross-linking agent can be expanded or shrunk according to the glucose concentration, and maintain the properties in the condition of pH 7.4 or less, and can form a gel state.

In the conditions, the gelling agent may be applicable, provided that the gelling agent is a biocompatible material which has biocompatibility and can cause gelatinization, which, for example, may be exemplified by biocompatible acrylamides. Specifically, they may include N-isopropylacrylamide (NIPAAm), N-isoprpylacrylamide, N,N-diethylacrylamide and N-isopropylmethacrylamide, etc.

In addition, as a cross-linking agent, similarly, biocompatible materials that can cause cross-link with a monomer may be applicable, which, for example, may be exemplified by N,N'-methylenebisacrylamide, ethyleneglycol dimethacrylate, N,N'-methylenebismethacrylamide and others.

Additionally, as a medicine, a peptide hormone such as insulin, a protein, an antibody, a nucleic acid and a variety of synthetic drugs may be used.

### (6) Various tests of the sugar-responsive gel

### (6-1) Changes in state of the sugar-responsive gel in response to glucose concentration

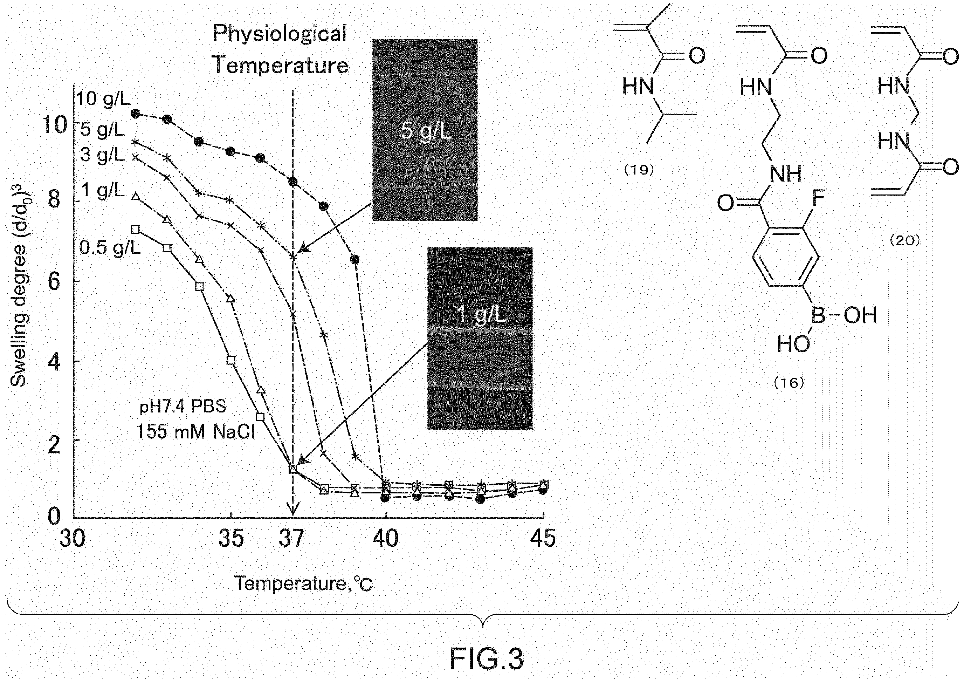

Next, as shown in Fig. 3, N-isopropylmethacrylamide (NIPMAAm) represented by the formula (19) serving as a gelling agent, the sample 1 (FPBA) as an example of the phenylboronic acids monomer of the present invention, and, N,N'-methylenebisacrylamide represented by the formula (20) serving as a cross-linking agent, were mixed in a molar ratio of 92.5/7.5/1, and thus the gel body in a state of gel of a cylindrical shape (1 mm diameter in dimethyl sulfoxide: DMSO as a reaction solvent) was produced. Then, the gel body was immersed in a pH 7.4 phosphate buffer solution (155 mM NaCl) containing 0.5 mg/l mL of insulin (derived from Bovine) modified with a fluorescein agent FITC (fluorescein isothiocyanate) for 24 hours at 4°C (degrees Celsius), and the FITC modified insulin was diffused into the gel body.

Next, the gel body was picked up from the phosphate buffer solution, immersed in 0.01 M hydrochloric acid for one hour at 37°C (degrees Celsius), so that a thin dehydrated shrunken layer (skin layer) was formed on the surface of the gel body, and thus a sugar-responsive gel with FITC modified insulin loaded in the gel body was produced.

Subsequently, the sugar-responsive gel was immersed in phosphate buffer solutions (155 mM NaCl) of pH 7.4, ion strength 0.15, prepared at 0.5 g/L, 1 g/L, 3g/L, 5g/L or 10 g/L of glucose concentrations, and then the temperatures of the phosphate buffer solutions with the respective glucose concentrations were adjusted at 34-45 °C (degrees Celsius). Then, the swelling degree (d/d₀)³ of each sugar-responsive gel at certain temperature and at each glucose concentration was measured. Thus, the results of the measurement shown in Fig.3 were obtained.

It should be noted that, the swelling degree referred to here, means a value obtained by calculating the ratio of d to d0 at each temperature and then cubing the ratio, wherein d0 is the diameter of the sugar-responsive gel in 0 g/L of glucose concentration in a phosphate buffer solution at each temperature, and d is the diameter of the sugar-responsive gel in the phosphate buffer solution at each temperature in a given glucose concentration. When the value of swelling degree is higher than 1, the value means that the sugar-responsive gel has caused swelling. When the value of swelling degree is lower than 1, the value means that the sugar-responsive gel has caused shrinking.

In the example, an image was taken by an optical microscope for each of the sugar-responsive gels immersed in the phosphate buffer solution prepared at 1 g/L of glucose concentration, and in the phosphate buffer solution prepared at 5 g/L of glucose concentration, at 37°C (degrees Celsius), respectively, and thus there could be obtained photographs showing that the swelling degree becomes larger as the glucose concentration increases, as shown in Fig. 3.

Additionally, it could be observed, from the graphs shown in Fig. 3 as well, that the swelling degree became larger as the glucose concentration increased at close to the temperature of the general physiological level (35-37°C (degrees Celsius)). These results showed that the sugar-responsive gel of the present invention could change its outer shape and cause swelling or shrinking in response to the level of glucose concentration at the physiological temperature level.

### (6-2) Controlled release of insulin in response to changes in glucose concentration

A verification test was conducted on controlled release of insulin responsive to changes in glucose concentration by use of the sugar-responsive gel produced according to the procedures described in the above-described section: "(6-1) Change in state of sugar-responsive gel in response to the concentration of glucose", wherein the sugar-responsive gel comprised the thin dehydrated layer (skin layer) formed on the surface of the gel body with FITC modified insulin loaded therein.

The verification test on the controlled release of insulin was conducted by high-performance liquid chromatography (HPLC). Specifically, phosphate buffer solution of pH 7.4 (155 mM NaCl) was used as a mobile phase (solvent), and the pump 1 was filled with the phosphate buffer solution prepared at 1 g/L concentration of glucose, while a pump 2 was filled with the phosphate buffer solution prepared at 5 g/L of glucose concentration, then the glucose concentration in the HPLC column was adjusted by successively changing or maintaining the mixing ratio thereof at 1 mL/min of flow speed.

Next, the sugar-responsive gel with FITC modified insulin loaded in the dehydrated shrunken layer was enclosed in the HPLC column filled with the phosphate buffer solution prepared at 1 g/L of glucose concentration, then placed in the HPLC flow path, and stabilized at the flow rate described above (1 mL/min) for 24 hours at 37°C (degrees Celsius).

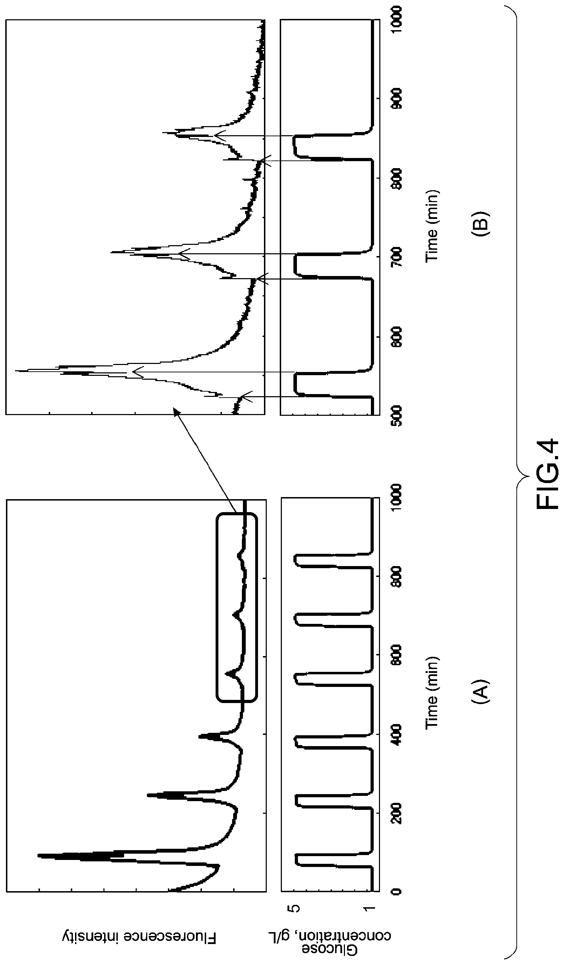

Subsequently, as shown in Fig.4A, the pump 1 and the pump 2 were controlled to repeat the changes in glucose concentration in the column from 1 g/L to 5 g/L, and from 5 g/L to 1 g/L at certain intervals, and thus the fluorescence intensity of the FITC modified insulin released from the sugar-responsive gel was measured (excitation and emission wavelengths were 495 nm and 520 nm, respectively). In the lower of the Fig. 4A are shown the glucose concentrations in the phosphate buffer solution flowed in the HPLC column, and these glucose concentrations represent practical values measured by the change in refraction index preliminarily calibrated for the phosphate buffer solution thereof.

In this manner, the glucose concentration was changed in the phosphate buffer solution at pH 7.4 and 37°C (degrees Celsius), and the amounts of the FITC modified insulin released from the sugar-responsive gel were observed based on the changes in fluorescence intensity. Then, it was observed that the fluorescence intensity was weak for 1 g/L of glucose concentration referred to as a normal blood-sugar level, and hence, it could be confirmed that the release of the FITC modified insulin from the sugar-responsive gel was suppressed. While, when the glucose concentration was 5 g/L, it was observed that the fluorescence intensity was sharply raised, and hence, it could be confirmed that the FITC modified insulin was released from the sugar-responsive gel.

Additionally, the relationships between the timing of rising of glucose concentration and fluorescence intensity, and the timing of dropping of glucose concentration and fluorescence intensity were investigated in further details, and thus, the results shown in Fig.4B were obtained. As shown in Fig. 4B, it was observed that the fluorescence intensity was sharply raised upon the rising of the glucose concentration, and when the increased glucose concentration was maintained at a constant level, the fluorescence intensity was gradually increased, and subsequently, reached a maximum value upon the dropping of the glucose concentration, and thereafter became gradually weak. Additionally, spikes, which implied the formation of the above described skin layer, were observed, synchronized with the timings of the changes in glucose concentrations.

From these results, it was found that: the sugar-responsive gel begins to release the FITC modified insulin sharply at substantially the same time as the rise in glucose concentration in the phosphate buffer solution; when the increased glucose concentration is maintained at the constant enhanced level, the release of the FITC modified insulin is allowed to continue; upon the start of dropping of the glucose concentration, the amount of the FITC modified insulin release reaches a maximum; and then followed by the gradual decrease in release of the FITC modified insulin as the glucose concentration decreases. It was also found that the FITC modified insulin loaded in the sugar-responsive gel was gradually decreased, from the fact that the peaks of fluorescence intensity became lower and lower with the repetition of the changes in glucose concentration between high-low levels.

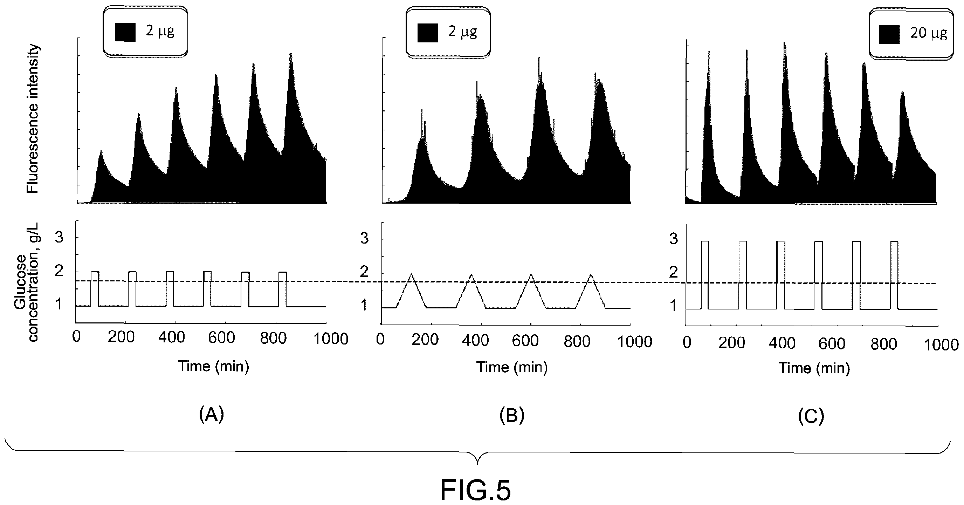

Next, the phosphate buffer solution prepared at 2 g/L of glucose concentration was freshly filled into the pump 2, and the glucose concentration in the HPLC column was adjusted by sequentially changing or maintaining the mixing ratio at 1 mL/min of flow rate. Practically, as shown in Fig.5A, the pump 1 and the pump 2 were controlled so as to repeat the changes in glucose concentration in the phosphate buffer solution at pH 7.4 and the temperature of 37°C (degrees Celsius) in the HPLC column between from 1 g/L to 2 g/L, and from 2 g/L to 1 g/L at a certain interval, and then the fluorescence intensity of the FITC modified insulin released from the sugar-responsive gel was measured. Herein, the glucose concentration of 1 g/L is equivalent to the normal blood-sugar level, and that of 2 g/L is equivalent to the diagnostic criteria level of diabetes mellitus.

In that case as well, it was observed from the level of the fluorescence intensity that the sugar-responsive gel began to release the FITC modified insulin, following the increase in the glucose concentration in the phosphate buffer solution, and that the amount of the released FITC modified insulin decreased gradually, following the decrease in the glucose concentration. Additionally, from the peaks of fluorescence intensity shown in Fig.4A and Fig.5A, it was found that the amounts of FITC modified insulin release per hour was increased according to the glucose concentration.

From these results, it was observed that the sugar-responsive gel did not release insulin at all when the glucose concentration was 1 g/L that is the normal blood-sugar level, and insulin was released when the glucose concentration was 2 g/L that is the diagnostic criteria level of diabetes mellitus, thus it is understood that the autonomous administration of insulin can be accomplished in response to the glucose concentration.

Next, as shown in Fig.5B, the pump 1 and the pump 2 were controlled to repeat the mild rising and mild dropping of the glucose concentration in the HPLC column from 1 g/L to 2 g/L, and from 2 g/L to 1 g/L at a certain interval, as in the similar manner described above, the fluorescence intensity of FITC modified insulin released from the sugar-responsive gel in the phosphate buffer solution at pH 7.4 and 37°C (degrees Celsius) was measured. Even in that case, it was observed that the release of the FITC modified insulin was increased according to the mild rising of glucose concentration, while the release of FITC modified insulin was decreased according to the mild dropping of glucose concentration.

In addition, the phosphate buffer solution of 3 g/L glucose concentration was freshly filled into the pump 2, the glucose concentration in the HPLC column was adjusted while changing or maintaining the mixing ratio sequentially at 1 mL/min of flow rate. Practically, as shown in Fig.5C, the pump 1 and the pump 2 were controlled to repeat the change in the glucose concentration from 1 g/L to 3 g/L, and from 3 g/L to 1 g/L, at a certain interval. In a similar manner to the above described, the fluorescence intensity of FITC modified insulin released from the sugar-responsive gel in the phosphate buffer solution at pH 7.4 and 37°C (degrees Celsius) was measured.

Also in that case, it could be observed that the release of FITC modified insulin was increased following the sharp rising of glucose concentration, while the release of FITC modified insulin was decreased following the sharp dropping of the glucose concentration. Additionally, when the glucose concentration was increased to 3 g/L that is the condition of the high blood-sugar level, compared with 2 g/L of the glucose concentration, the release of insulin per hour was increased from 2 µg (microgram) to 20 µg (microgram) that was about 10 times increase.

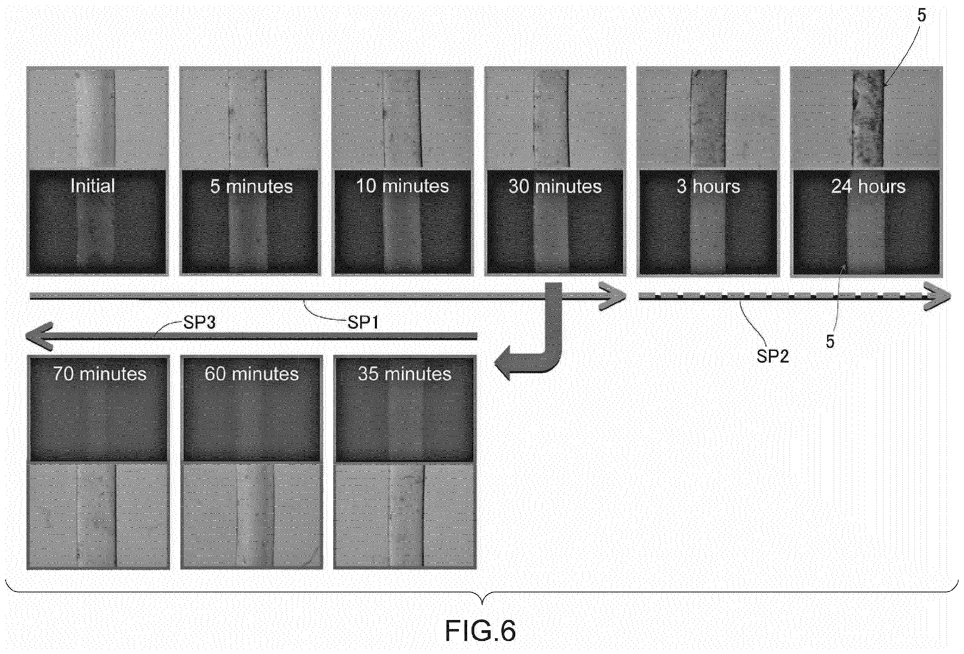

As described above, in the controlled release of insulin by utilizing on the gel, it has been considered that a reversible formation of skin layer has an important role. For ascertaining this consideration directly, the experiment described below was conducted. The sugar-responsive gel formed in a cylindrical shape was immersed in the phosphate buffer solution prepared at 2 g/L of glucose concentration, pH 7.4 and 37°C (degrees Celsius), and the glucose concentration was decreased from 2 g/L to 1 g/L, then the states were observed at 5 min, 10 min, 30 min, 3 hours, 24 hours after (the step SP 1 and step SP 2). Fig.6 shows the images of the sugar-responsive gel 5 by transmitted light at the upper, and the fluorescence images emitted from the sugar-responsive gel 5 at the lower.

In that case, 8-anilino-1-naphtalene-sulfonic acid was used as a fluorescence probe molecule for the FITC modified insulin diffused to the gel body of the sugar-responsive gel. This reagent has a property to change a fluorescence intensity remarkably in response to change in electric permittivity at a local environment. That means, it is possible to visualize the environment of low electric permittivity as the increase in fluorescence intensity following the formation of the skin layer.

As the results shown in Fig.6, it was observed that the sugar-responsive gel 5 gradually increased the fluorescence intensity on the surface and the skin layer on the surface of the gel has formed, when certain period has passed after reduction of the glucose concentration from 2 g/L to 1 g/L.

Additionally, as another example, the sugar-responsive gel 5 formed in a cylindrical shape was immersed in the phosphate buffer solution at pH 7.4 and 37°C (degrees Celsius) prepared at 2 g/L of glucose concentration, and as shown in Fig.6, the glucose concentration in the phosphate buffer solution was reduced from 2 g/L to 1 g/L (step SP 1), and 30 min after, the glucose concentration was recovered from 1 g/L into 2 g/L again, then the states at 35 min, 60 min and 70 min after, were observed (step SP3).

As the results shown in Fig.6, regarding the sugar-responsive gel 5, it was observed as follows; when the glucose concentration was reduced to 1 g/L, the fluorescence intensity on the surface of the gel became strong gradually, then, by recovering the glucose concentration to 2 g/L again, the fluorescence intensity on the surface gradually became weak, the skin layer on the surface of the gel disappeared rapidly.

### (7) Effects and advantages

In the above embodiments, since the sugar-responsive gel contains a phenylboronic acids having fluorophenylboronic acids group wherein a single of or a plurality of fluorine is substituted for hydrogens on the phenyl ring as represented by the above formula (5), pKa can be controlled to pH 7.4 or less of physiological level.

Additionally, for the sugar-responsive gel, a gel body has been configured to be formed according to copolymerization with a gelling agent via unsaturated bond of phenylboronic acids, and insulin is diffused to the gel body, the surface of the gel body is enclosed with a dehydrated shrunken layer. According to this configuration, the sugar-responsive gel can release insulin from inner part to outer part of the gel body, when the glucose concentration is increased at pKa 7.4 or less and 35-40°C (degrees Celsius) of the physiological condition, then the dehydrated layer is disappeared according to the swelling of gel body.

While, the sugar-responsive gel, when the glucose concentration decreases from the condition again, can be suppressed releasing insulin from inner part to outer part of the gel body by reformation of the dehydrated layer (skin layer) on all of the surface by shrinking of the expanded state of the gel body.

Accordingly, the sugar-responsive gel of the present invention can release insulin autonomously in response to the glucose concentration, thus, the sugar-responsive gel has pKa more suitable for utilization in the physiological environment than convention, and can be applied for autonomous administration of insulin.

### (8) Insulin administering device of a first embodiment

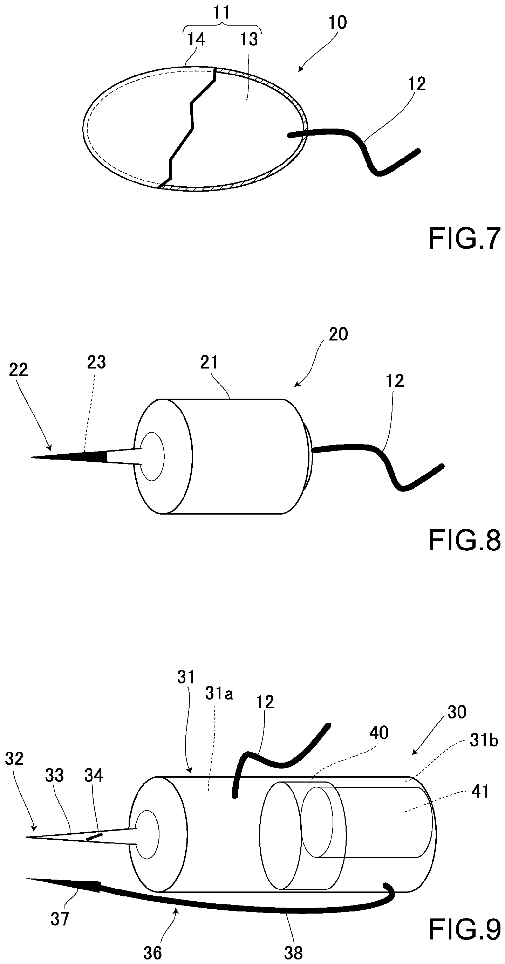

Next is a description of an insulin administering device utilizing the sugar-responsive gel described above. In Fig. 7, numeral 10 shows an insulin administering device according to a first embodiment, a method for implantation into a certain site in the body of the subject to be administered with a medicine is applicable. Practically, this insulin administering device 10 is configured as that the insulin administering control unit 11 is implanted into the body of a patient, insulin is supplied from out of the patient body to the insulin administering control unit 11 via a supplying unit 12 as the occasion demands.

Insulin administering control unit 11 comprises the sugar-responsive gel 13, and the enclosing unit 14 which is formed to cover all of the surface of the sugar-responsive gel 13, further, a supplying unit 12 in a tube state is connected to the sugar-responsive gel 13, and insulin can be supplied from out to the sugar-responsive gel 13 as the occasion demands. The sugar-responsive gel 13 is the described above "(4) a sugar-responsive gel comprising phenylboronic acids monomer", comprising a gel body wherein insulin is diffused, and a dehydrated shrunken layer enclosing the gel body. By this configuration, when the glucose concentration is increased in a patient and the blood sugar level become high, the sugar-responsive gel 13 can release insulin in the gel body by reacting with glucose in response to the increase in glucose concentration and elevation of the blood-sugar level.

The enclosing unit 14 that covers all of the surface of the sugar-responsive gel 13 as a releasing unit, has biocompatibility such as polyethylene glycol and the like, and is made of a biocompatible material through which glucose and insulin can pass. The enclosing unit 14 encloses the sugar-responsive gel 13, so as to protect the sugar-responsive gel 13 not to cause a diffusion via disruption by outer pressure.

In the configuration described above, the insulin administering device 10 is implanted, wherein the insulin administering control unit 11 having the sugar-responsive gel 13 containing phenylboronic acids monomer represented by the formula (5) described above is embedded into the body of a patient, and the supplying unit 12 is drawn out to out of the body.

In this manner, in the insulin administering device 10, when the glucose concentration level of a patient to be administered with the medicine is increased, insulin can be released from the inner sugar-responsive gel 13 in response to increase in the glucose concentration, while, when the glucose concentration is decreased, the release of insulin from the sugar-responsive gel 13 can be suppressed in response to the decrease in the glucose concentration. Then, autonomous administration of insulin in response to the glucose concentration can be carried out.

Additionally, in the insulin administering device 10, a supplying unit 12 is connected to the sugar-responsive gel 13 in the insulin administering control unit 11, insulin can be supplied to the sugar-responsive gel 13 embedded in the body from the supplying unit 12. In this manner, in the insulin administering device 10, if insulin causes deficiency in the sugar-responsive gel 13, the complement of insulin can be conducted from the out of the body without picking out the insulin administering control unit 11 from the body, thus the insulin administering device 10 can be used for a long time.

### (9) An insulin administering device of a second embodiment

In the Fig. 8, numeral 20 shows an insulin administering device of the second embodiment. The insulin administering device 20 can utilize a way of the indwelling needle 22 which is inserted into the skin of the body and capable of supplying insulin from the out of the body. Practically, the insulin administering device 20 comprises the indwelling needle 22, and an insulin filling unit 21 integrally formed with the indwelling needle 22, an supplying unit 12 connected to the insulin filling unit 21. Additionally, the described above "(4) sugar-responsive gel containing phenylboronic acids monomer" is filled into the inner space of the indwelling needle 22.

The indwelling needle 22 is formed whose tip is a shape of needle in a conical shape, and by insertion of the tip into the skin of a patient, the sugar-responsive gel 23 can be contacted with the patient blood with the sugar-responsive gel 23 in the inner space. By this configuration, the sugar-responsive gel 23 can release insulin from the gel body in response to the blood glucose concentration, and it is formed so that the insulin is administered into the patient body from the inner part of indwelling needle 22.

Furthermore, in this embodiment, the embodiment of the small inner part in a conical shape in the indwelling needle 22 is described as the sugar-responsive gel 23 is filled therein, however, the present invention is not limited to the embodiment. The sugar-responsive gel 23 may be filled into the other area, provided that the sugar-responsive gel 23 in the indwelling needle 22 is certainly contacted with the blood by insertion of the indwelling needle 22 into the patient skin and that insulin can be released from the gel body in response to the blood glucose concentration.

Additionally, the indwelling needle 22 is formed so that the inner part of the body side thereof has a connection with the inner part of the insulin filling unit 21 to supply insulin in the insulin filling unit 21 to the sugar-responsive gel 23. By this configuration, the sugar-responsive gel 23 can maintain the state of containing insulin, even if it is filled into the micro-region of the indwelling needle 22.

While, the insulin filling unit 21, whose inner space is filled with insulin via the supplying unit 12, can supply insulin to the sugar-responsive gel 23 in the indwelling needle 22. By this way, the insulin administering device 20 can always supply insulin complementarily to the sugar-responsive gel 23 from the insulin filling unit 21, even if insulin is released from the sugar-responsive gel 23.

In the above configuration, the insulin administering device 20 is configured to comprise the sugar-responsive gel 23 containing phenylboronic acids monomer represented by the above formula (5) in the indwelling needle 22, the tip of the indwelling needle 22 is hold with insertion through the skin of a patient, then the patient blood is contacted with the sugar-responsive gel 23.

By this way, in the insulin administering device 20, insulin can be released from the sugar-responsive gel 23 in response to the increase in patient glucose concentration, while insulin release from the sugar-responsive gel 23 in the indwelling needle 22 can be suppressed in response to the decrease in the glucose concentration. Thus, the autonomous administration of insulin in response to the glucose concentration can be carried out.

### (10) Insulin administering device of a third embodiment

In Fig.9, 30 shows an insulin administering device of the third embodiment comprising: an indwelling needle with backflow prevention valve 32 having a backflow prevention valve 34 in the indwelling needle 33; an insulin filling unit 31 with pumping function integrally formed in the indwelling needle with backflow prevention valve 32 to the body side; a supplying unit 12 that supplies insulin to an insulin filled area 31a of an insulin filling unit with pumping function 31; and, an introducing unit 36 for introducing the patient blood to the pumping region 31b in the insulin filling unit with pumping function 31.

The indwelling needle with backflow prevention valve 32 leads a flow of insulin in the insulin filling unit 31 a to the front side, only when a pressure from insulin fulfilling unit with pumping function 31 in the body side to the front side is elevated over a predetermined level, while the flow from the front side to the body side is prevented by the backflow prevention valve 34 placed in the inner part of the conical-shaped indwelling needle 33.

The inner part of the insulin filling unit with pumping function 31 is separated into an insulin filling region 31 a where insulin is filled and a pumping region 31b where blood is supplied, by a plate-like separator part 40. Insulin can be filled into the insulin filling region 31a in the insulin filling unit with pumping function 31 via a supplying unit 12 comprising a tube. While, in the pumping region 31b of the insulin filling unit with pumping function 31, a cylindrically-shaped sugar-responsive gel 41 is disposed so as to be contacted with the edge part and the separator part 40, and thus, the patient blood is supplied by an introducing unit 36, and the blood can make a contact with the sugar-responsive gel 41.

Here, the sugar-responsive gel 41 comprises a gel body containing a phenylboronic acids monomer represented by the formula (5) in a similar manner with the described above "(4) sugar-responsive gel containing phenylboronic acids monomer". However, in the sugar-responsive gel 41 in this embodiment, insulin has not been diffused in the gel body and a dehydrated and shrunken layer has not been formed on the surface of the gel body unlike in the one described above. That is, the sugar-responsive gel 41 does not release insulin in response to the glucose concentration, and has a configuration wherein swelling and shrinking can merely occur.

As for the introducing unit 36 connected to the pumping region 31b, the tip of the needle disposed in the front of the indwelling needle 37 is inserted into the patient skin, and the blood is introduced to the pumping region 31b of the insulin filing unit with pumping function 31 from the indwelling needle 37 through the tube 38.

By this way, the insulin filling unit with pumping function 31 is configured so that the sugar-responsive gel 41 can cause swelling or shrinking in response to the blood glucose concentration by contacting the sugar-responsive gel 41 with the blood. By this configuration, regarding the insulin filling part with pumping function 31, for example, when blood glucose concentration is increased, the sugar-responsive gel 41 causes expansion in response to the glucose concentration, and the separator part 40 is moved by a pressure from the sugar-responsive gel 41, then the insulin filling region 41a can be made smaller. According to the way, the insulin filling region with pumping function 31 can supply insulin in the insulin filling region 31a to the indwelling needle with backflow prevention valve 32, and insulin can be administered from the indwelling needle with backflow prevention valve 32 into the patient body.

While, as for the insulin filling unit with pumping function 31, for example, when the blood glucose concentration is decreased, the sugar-responsive gel 41 causes shrinking in response to the glucose concentration, the pressure caused by the separator part 40 due to the sugar-responsive gel 41 is stopped, then the administration of insulin in the insulin filling region 31a from the indwelling needle with backflow prevention valve 32 to the patient body can be discontinued.

As for the configuration described above, the insulin administering device 30 comprises the sugar-responsive gel 41 containing phenylboronic acids monomer represented by the described above formula (5) in the pumping region 31b of the insulin filling unit with pumping function 31, the tip of the indwelling needle with backflow prevention valve 32 and the tip of the indwelling needle 37 are inserted through the skin of the patient and held. While, in the insulin administering device 30, the patient blood is introduced into the pumping region 31b of the insulin filling unit with pumping function 31 by the introducing unit 36, and blood is contacted with the sugar-responsive gel 41.

By this way, in the insulin administering device 30, when the patient glucose concentration is increased, the sugar-responsive gel 41 in the pumping region 31b responds to this and causes expansion of the sugar-responsive gel 41 in the pumping region 31b, and the separator part 40 is moved to the insulin filling region 31a side by the expansion pressure, thus insulin in the insulin filling region 31a can be released into the body from the indwelling unit with backflow prevention valve 32. Additionally, in the insulin administering device 30, when the glucose concentration is decreased, the sugar-responsive gel 41 responds and causes shrinking, then the separator part 40 stops moving to the filling region 31a side, and thus the insulin release into the body can be suppressed. Thus, in the insulin administering device 30, insulin can be autonomously administered in response to the glucose concentration.

While, the present invention should not be limited to the foregoing embodiments, a variety of modifications can be applied within a range of the scope of the present invention. For example, as any ratio of the polymerization between N-isopropylmethacrylamide (NIPMAAm) and the example sample 1 phenylboronic acids monomer of the present invention (FPBA), a variety of the molar ratios may be prepared within a range of 90/10 to 70/30 and the like, further any other variety of ratios can be applied. In these embodiments, around the normal physiological temperature (35°C - 37°C), the prominent swelling can be caused in response to the glucose concentration, thus, the remarkable effects can be achieved sufficiently for a sugar-responsive gel which can be utilized for administering insulin autonomously.

Additionally, in the embodiments described above, glucose is applied as a sugar, however, in the present invention, not limited to this, other sugars that have a structure of 1,2-diol, or 1,3-diol such as galactose, mannose or fructose may be applied.

## Claims

1. A device for administering a medicine in response to a blood-sugar level of a subject to be administered with a medicine, comprising:
a sugar-responsive gel, comprising a gel body and a dehydrated shrunken layer enclosing the gel body, wherein a medicine is contained in the gel body, wherein the gel body and the dehydrated shrunken layer are formed by a gel composition containing a gelling agent, a cross-linking agent and a phenylboronic acids monomer represented by the following formula (1): 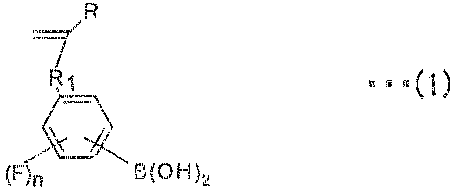 wherein:
R is H or CH₃;
F is independently present;
n is 1, 2, 3 or 4; and
R₁ is a divalent linking group.
an introducing unit for introducing the blood of the subject to be administered with the medicine into the sugar-responsive gel; and
a releasing unit for releasing the medicine into the subject to be administered with the medicine based on a change in the sugar-responsive gel in response to a blood-sugar level of the blood,
wherein the introducing unit and the releasing unit are an indwelling needle whose tip is inserted into the administration site to be held therein, and the sugar-responsive gel is filled into the indwelling needle.

2. A device for administering a medicine in response to a blood-sugar level of a subject to be administered with a medicine, comprising:
a sugar-responsive gel, comprising a gel body, wherein a medicine is contained in the gel body, wherein the gel body is formed by a gel composition containing a gelling agent, a cross-linking agent and a phenylboronic acids monomer represented by the following formula (1): 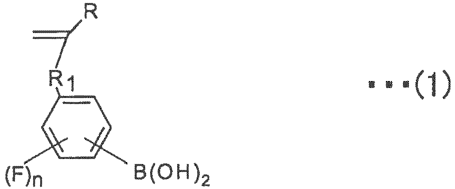 wherein:
R is H or CH₃;
F is independently present;
n is 1, 2, 3 or 4; and
R₁ is a divalent linking group;
an introducing unit for introducing the blood of the subject to be administered with the medicine into the sugar-responsive gel;
a releasing unit for releasing the medicine into the subject to be administered with the medicine based on a change in the sugar-responsive gel in response to a blood-sugar level of the blood;
an indwelling needle with a backflow prevention valve whose tip is inserted into the administration site to be held therein; and
a filling unit filled with the medicine and communicated with the indwelling needle with the backflow prevention valve,
wherein the releasing unit releases the medicine into the administration site from the indwelling needle with the backflow prevention valve in such a manner that the medicine in the filling unit is pushed out to the indwelling needle with the backflow prevention valve by an expansion pressure generated in response to the blood-sugar level of the blood introduced by the introducing unit.

3. The medicine administering device of claim 1 or 2, comprising a supplying unit for supplying the medicine to the sugar-responsive gel.

## Patentansprüche

1. Vorrichtung zum Verabreichen eines Arzneimittels als Reaktion auf einen Blutzuckerspiegel eines Patienten, dem ein Arzneimittel verabreicht wird, umfassend:
ein zuckerempfindliches Gel, umfassend einen Gelkörper und eine dehydrierte geschrumpfte Schicht, die den Gelkörper umschließt, wobei ein Arzneimittel in dem Gelkörper enthalten ist, wobei der Gelkörper und die dehydrierte geschrumpfte Schicht durch eine Gelzusammensetzung gebildet sind, die ein Geliermittel, ein Vernetzungsmittel und ein durch die folgende Formel (1) dargestelltes Phenylboronsäuremonomer umfasst: 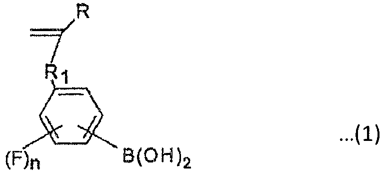 wobei:
R H oder CH₃ ist;
F unabhängig vorhanden ist;
n 1, 2, 3 oder 4 ist; und
R1 eine bivalente Verbindungsgruppe ist;
eine Einführungseinheit zum Einführen des Blutes des Patienten, dem ein Arzneimittel verabreicht wird, in das zuckerempfindliche Gel; und
eine Freisetzungseinheit zum Freisetzen des Arzneimittels in dem Patienten, dem ein Arzneimittel verabreicht wird, basierend auf einer Veränderung des zuckerempfindlichen Gels als Reaktion auf einen Blutzuckerspiegel des Blutes,
wobei sowohl die Einführungseinheit als auch die Freisetzungseinheit eine Verweilnadel sind, deren Spitze in die dort zu haltende Verabreichungsstelle eingeführt ist, und das zuckerempfindliche Gel in die Verweilnadel gefüllt ist.

2. Vorrichtung zum Verabreichen eines Arzneimittels als Reaktion auf einen Blutzuckerspiegel eines Patienten, dem ein Arzneimittel verabreicht wird, umfassend:
ein zuckerempfindliches Gel, umfassend einen Gelkörper, wobei ein Arzneimittel in dem Gelkörper enthalten ist, wobei der Gelkörper durch eine Gelzusammensetzung gebildet ist, die ein Geliermittel, ein Vernetzungsmittel und ein durch die folgende Formel (1) dargestelltes Phenylboronsäuremonomer umfasst: 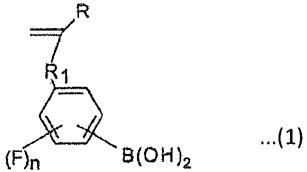 wobei:
R H oder CH₃ ist;
F unabhängig vorhanden ist;
n 1, 2, 3 oder 4 ist; und
R1 eine bivalente Verbindungsgruppe ist;
eine Einführungseinheit zum Einführen des Blutes des Patienten, dem ein Arzneimittel verabreicht wird, in das zuckerempfindliche Gel;
eine Freisetzungseinheit zum Freisetzen des Arzneimittels in dem Patienten, dem ein Arzneimittel verabreicht wird, basierend auf einer Veränderung des zuckerempfindlichen Gels als Reaktion auf einen Blutzuckerspiegel des Blutes,
eine Verweilnadel mit einem Rückflussverhinderungsventil, dessen Spitze in die dort zu haltende Verabreichungsstelle eingeführt ist; und
eine Fülleinheit, die mit dem Arzneimittel gefüllt und mit der Verweilnadel mit dem Rückflussverhinderungsventil verbunden ist,
wobei die Freigabeeinheit das Arzneimittel in die Verabreichungsstelle aus der Verweilnadel mit dem Rückflussverhinderungsventil derart freigibt, dass das Arzneimittel in der Fülleinheit aus der Verweilnadel mit dem Rückflussverhinderungsventil durch einen Expansionsdruck herausgedrückt ist, der als Reaktion auf den Blutzuckerspiegel des durch die Einführungseinheit eingeführten Blutes entsteht.

3. Arzneimittelverabreichungsvorrichtung nach Anspruch 1 oder 2, umfassend eine Zuführeinheit zum Zuführen des Arzneimittels in das zuckerempfindlichen Gel.

## Revendications

1. Dispositif d'administration d'un médicament en réponse à une glycémie d'un sujet auquel un médicament doit être administré, comprenant :
un gel sensible au sucre, comprenant un corps de gel et une couche réduite déshydratée entourant le corps de gel, un médicament étant contenu dans le corps de gel, le corps de gel et la couche réduite déshydratée étant formés d'une composition de gel contenant un agent gélifiant, un agent de réticulation et un monomère d'acides phénylboriques représenté par la formule (1) suivante : 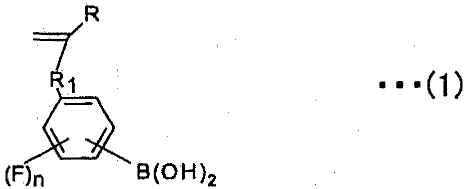 dans laquelle :
R est H ou CH₃ ;
F est indépendamment présent ;
n est 1, 2, 3 ou 4 ; et
R₁ est un groupe de liaison divalent,
une unité d'introduction pour introduire le sang du sujet auquel le médicament doit être administré dans le gel sensible au sucre ; et
une unité de libération pour libérer le médicament dans le sujet auquel le médicament doit être administré sur la base d'un changement dans le gel sensible au sucre en réponse à une glycémie du sang,
dans lequel l'unité d'introduction et l'unité de libération sont une aiguille à demeure dont la pointe est insérée dans le site d'administration de manière à être maintenue dans celui-ci, et le gel sensible au sucre est chargé dans l'aiguille à demeure.

2. Dispositif d'administration d'un médicament en réponse à une glycémie d'un sujet auquel un médicament doit être administré, comprenant :
un gel sensible au sucre, comprenant un corps de gel, un médicament étant contenu dans le corps de gel, le corps de gel étant formé d'une composition de gel contenant un agent gélifiant, un agent de réticulation et un monomère d'acides phénylboriques représenté par la formule (1) suivante : 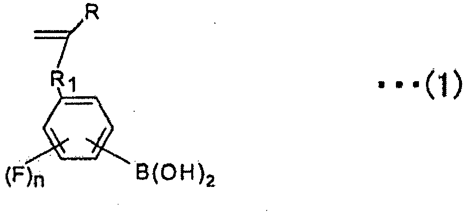 dans laquelle
R est H ou CH₃ ;
F est indépendamment présent ;
n est 1, 2, 3 ou 4 ; et
R₁ est un groupe de liaison divalent ;
une unité d'introduction pour introduire le sang du sujet auquel le médicament doit être administré dans le gel sensible au sucre ; et
une unité de libération pour libérer le médicament dans le sujet auquel le médicament doit être administré sur la base d'un changement dans le gel sensible au sucre en réponse à une glycémie du sang ;
une aiguille à demeure avec une valve de prévention de refoulement dont la pointe est insérée dans le site d'administration de manière à être maintenue dans celui-ci ; et
une unité de remplissage remplie du médicament et communiquant avec l'aiguille à demeure avec la valve de prévention de refoulement,
dans lequel l'unité de libération libère le médicament dans le site d'administration depuis l'aiguille à demeure avec la valve de prévention de refoulement de telle manière que le médicament dans l'unité de remplissage soit expulsé vers l'aiguille à demeure avec la valve de prévention de refoulement par une pression d'expansion générée en réponse à la glycémie du sang introduit par l'unité d'introduction.

3. Dispositif d'administration de médicament de la revendication 1 ou 2, comprenant une unité de distribution pour distribuer le médicament vers le gel sensible au sucre.
